**Europäisches Patentamt**

**European Patent Office**

**Office européen des brevets**

(11) Veröffentlichungsnummer: **0 082 251
B1**

(12) # EUROPÄISCHE PATENTSCHRIFT

(45) Veröffentlichungstag der Patentschrift:
**05.08.87**

(51) Int. Cl.⁴: **A 61 M 5/18**

(21) Anmeldenummer: **82108317.7**

(22) Anmeldetag: **09.09.82**

(54) Injektionsspritze für zwei Flüssigkeiten.

(30) Priorität: **22.12.81 CH 8184/81**

(43) Veröffentlichungstag der Anmeldung:
**29.06.83 Patentblatt 83/26**

(45) Bekanntmachung des Hinweises auf die Patenterteilung:
**05.08.87 Patentblatt 87/32**

(84) Benannte Vertragsstaaten:
**CH DE FR GB IT LI NL SE**

(56) Entgegenhaltungen:
**CH - A - 440 560
CH - A - 445 721
CH - A - 580 427
DE - C - 730 362
GB - A - 1 214 053
US - A - 3 685 514**

(73) Patentinhaber: **CONTRAVES AG,
Schaffhauserstrasse 580, CH-8052 Zürich (CH)**

(72) Erfinder: **Gähwiler, Hermann, Köschenrütistrasse 3,
CH-8052 Zürich (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft eine Injektionsspritze zum aufeinanderfolgenden Einspritzen von zwei Flüssigkeiten in Blutgefässe lebender Körper, bestehend aus einem auf der einen Seite mit einem Befestigungsflansch und auf der anderen Seite in der Nähe einer Austrittsöffnung mit einem Katheteransatz versehenen Zylinder, in welchem ein erster Kolben und ein zweiter Kolben relativ zueinander sind, wobei die Kolben durch mit ihnen verbundenen und ineinandergesteckten Kolbenstangen betätigbar sind.

Aus der DE-AS 730 363 ist zum Injizieren verschiedener Spritzmittel eine derartige mit zwei in einem Zylinder hintereinanderliegenden Kolben versehene Injektionsspritze bekannt, welche seitlich an dem Zylinder mit einer bypassartig ausgebildeten Abzweigung versehen ist, die mit zwei übereinanderliegenden Öffnungen mit dem Zylinderinnern in Verbindung steht. Die in dem Zylinder verschiebbaren Kolben sind je mit einer Kolbenstange derart wirkverbunden, dass die beiden ineinandergesteckten Kolbenstangen je nach Stellung des einzelnen Kolbens in bezug auf die Öffnungen getrennt mittels einem knopfartigen Handgriff oder durch seitlich angeordnete Griffe betätigbar sind.

Insbesondere in der Computertomographie gewinnt eine Injektionsmethode an Bedeutung, bei der unmittelbar nach der Kontrastmittelinjektion eine physiologische Kochsalzlösung eingespritzt wird. Mit einer weiterhin bekannten Injektionsspritze gemäss der CH-PS 580 427, kann in einem Arbeitsgang jedoch nur eine Flüssigkeit injiziert werden. Es müssen deshalb jeweils zwei mit je einer Flüssigkeit gefüllte Spritzen, die je mit einem Antrieb versehen sind, eingesetzt werden. Neben den in einem Arbeitsgang jedoch nur eine Flüssigkeit injiziert werden. Es müssen deshalb jeweils zwei mit je einer Flüssigkeit gefüllte Spritzen, die je mit einem Antrieb versehen sind, eingesetzt werden. Neben den hohen Kosten für den Einsatz von zwei Spritzen und Antrieben erfordert dies einen hohen Aufwand für die Bedienung und Überwachung.

Der Erfindung liegt die Aufgabe zugrunde, eine Injektionsspritze zum aufeinanderfolgenden Injizieren von zwei Flüssigkeiten zu schaffen, die einfach im Aufbau und kostengünstig herstellbar ist, und welche die hohen Anforderungen bezüglich Zuverlässigkeit und Genauigkeit erfüllt, die an solche medizinischen Geräte gestellt werden.

Die Aufgabe wird dadurch gelöst, dass die mit dem einen Ende mit dem zweiten Kolben verbundene Kolbenstange als Distanzstange ausgebildet und mit dem anderen Ende in einer axialen Bohrung des ersten Kolbens durch eine federelastische Haltevorrichtung derart gehalten ist, dass nach dem Ausstossen der zwischen zweitem Kolben und der Austrittsöffnung befindlichen Flüssigkeit durch das Zusammenwirken des zweiten Kolbens mit der Zylinderkante des Zylinderbodens die Haltevorrichtung selbsttätig gelöst und dadurch der erste Kolben relativ zu dem zweiten Kolben zum Ausstossen der zwischen erstem und zweitem Kolben befindlichen Flüssigkeit verschiebbar ist.

Weitere erfindungsgemässe Ausbildungen ergeben sich aus den abhängigen Ansprüchen.

Erfindungsgemässe Ausführungsbeispiele werden nun im folgenden anhand der Zeichnung beschrieben. Es zeigen:

Fig. 1 eine teilweise im Schnitt dargestellte Injektionsspritze vor einer Injektion,

Fig. 2 eine Injektionsspritze während dem Ausstossen der zwischen erstem und zweitem Kolben befindlichen Flüssigkeit,

Fig. 3 eine Injektionsspritze nach beendeter Injektion,

Fig. 4 eine Injektionsspritze mit einem Trennkolben nach einer weiteren erfindungsgemässen Variante, während dem Ausstossen der zwischen erstem Kolben und Austrittsöffnung befindlichen Flüssigkeit, und

Fig. 5 während dem Ausstossen der zwischen erstem und zweitem Kolben befindlichen Flüssigkeit, sowie

Fig. 6 eine Draufsicht auf den Trennkolben.

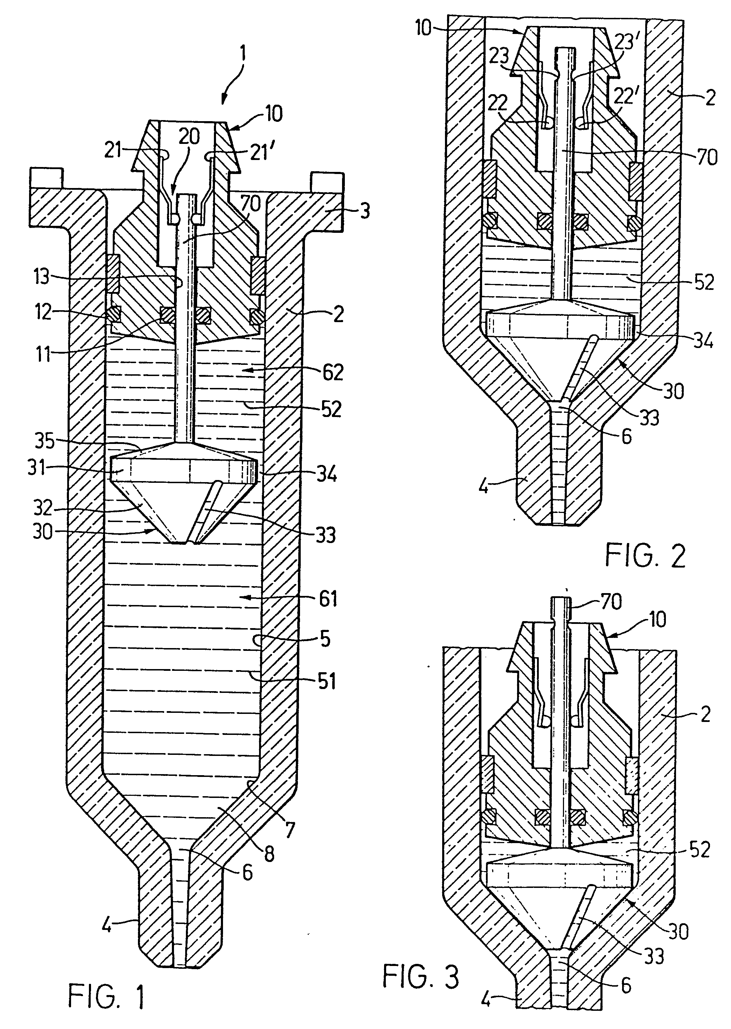

In Fig. 1 ist eine Injektionsspritze 1 mit einem Zylinder 2, einem Kolben 10, einem Befestigungsflansch 3 sowie einem Ansatzteil 4 für ein Katheter dargestellt, die einsatzbereit mit zwei Flüssigkeiten 51 und 52 gefüllt ist. Die erste Flüssigkeit 51, beispielsweise Kontrastmittelflüssigkeit, befindet sich in einer vorderen Kammer 61, während die zweite Flüssigkeit 52, beispielsweise Spülflüssigkeit, sich in einer hinteren Kammer 62 befindet. Ein Trennkolben 30 mit einer konischen Frontfläche 32, einer konischen Rückfläche 35 und einer zylindrischen Mantelfläche 31 trennt beide Flüssigkeiten 51 und 52. Zwischen Mantelfläche 31 des Trennkolbens 30 und Zylinderinnenwand 5 besteht ein zylindrischer Spalt 34, der eine kleine Breite, beispielsweise 1 mm, aber eine grosse Höhe, beispielsweise 15 mm, aufweist, so dass die Vermischung der beiden Flüssigkeiten 51 und 52 vernachlässigbar klein ist. Am Trennkolben 30 ist eine Distanzierstange 70 befestigt, die flüssigkeitsdicht durch eine Bohrung 13 des Kolbens 10 führt, und mittels einer Haltevorrichtung 20 mit diesem lösbar verbunden ist. Die Haltevorrichtung 20 besteht beispielsweise aus zwei Rastfedern 21 und 21' mit je einem Rastnocken 22 und 22', die in je eine Vertiefung 23 und 23' der Distanzierstange 70 eingreifen.

Eine weitere Möglichkeit, den Trennkolben 30 während dem Ausstossen der ersten Flüssigkeit 51 am Kolben 10 gegen Verschiebung in axialer Richtung zu sichern, wird durch starke Reibung der Distanzierstange 70 in der Bohrung 13 des Kolbens 10, beispielsweise durch entsprechend enge Bohrung, erreicht. Dies hat den Vorteil, dass der Abstand des Trennkolbens 30 zum Kolben 10 kontinuierlich verändert werden kann, und damit das Volumen der hinteren Kammer 62 veränderlich ist.

Zur Injektion der ersten Flüssigkeit 51 wird der Kolben 10 und der Trennkolben 30, der durch die Distanzierstange 70 in konstantem Abstand gehalten wird, zur Austrittsöffnung 6 hin verschoben, bis die Frontfläche 32 des Trennkolbens 30 den konischen Zylinderboden 8 erreicht. Die erste Flüssigkeit 51 ist jetzt ausgestossen (Fig. 2), die zweite Flüssigkeit 52 aber noch vollständig in der hinteren Kammer 62 vorhanden. Zur Injektion der zweiten Flüssigkeit 52 wird nun die Haltevorrichtung 20 durch verstärkten Druck auf den Kolben 10 durch Ausrasten der Rastfedern 21 und 21′ gelöst, wodurch der Trennkolben 30 zum Kolben 10 hin verschiebbar wird. Durch Vorschieben des Kolbens 10 wird die zweite Flüssigkeit 52 durch den zylindrischen Spalt 34 zur Austrittsöffnung 6 hin ausgestossen. In der Fig. 2 ist die Injektionsspritze im Zustand nach dem Ausstossen der ersten Flüssigkeit und nach teilweisem Ausstossen der zweiten Flüssigkeit und in der Fig. 3 nach dem Ausstossen beider Flüssigkeiten dargestellt.

Um ein Durchfliessen der zweiten Flüssigkeit nach dem Ausstossen der ersten Flüssigkeit zu gewährleisten, sind auf der Frontfläche 32 des Trennkolbens 30 Durchflusskanäle 33 vorgesehen. Versuche haben jedoch gezeigt, dass insbesondere bei der Verwendung von physiologischer Kochsalzlösung als Spülmittel, auch ein Trennkolben ohne Durchflusskanäle keine Verstopfungen im Bereich der Austrittsöffnung verursacht.

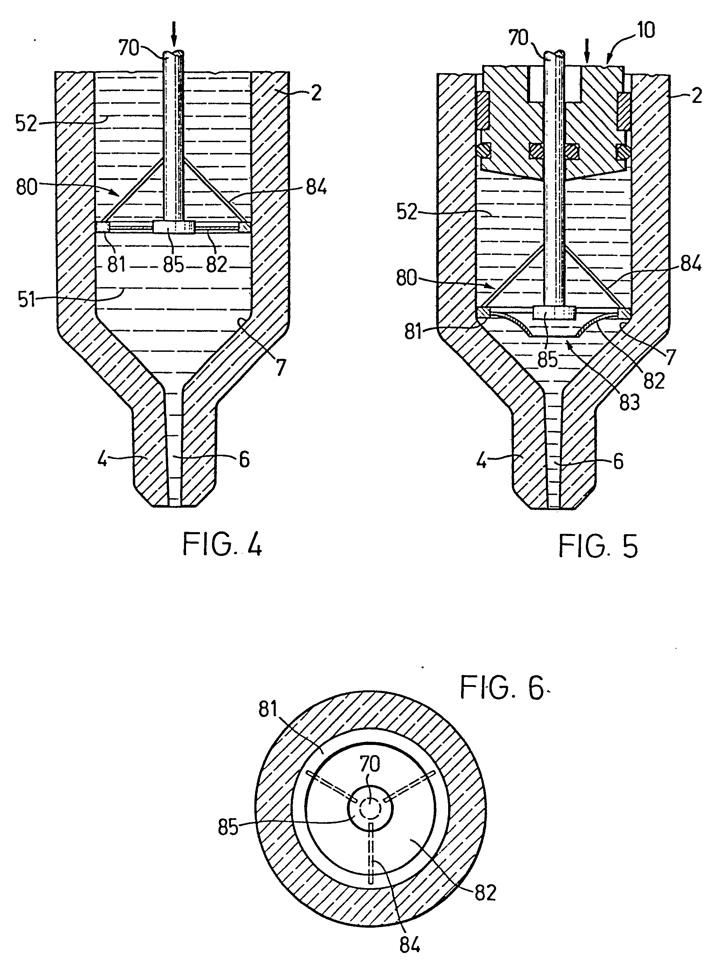

In den Fig. 4 und 5 ist eine weitere Variante eines Trennkolbens 80 dargestellt, der einen Stützring 81, eine daran befestigte biegsame Lamelle 82, sowie ein zentrales, an der Distanzierstange 70 befestigtes Verschlussteil 85 aufweist. Der Stützring 81 wird durch mehrere – beispielsweise drei – seitliche, an der Distanzierstange 70 befestigte Streben 84 gehalten. Während dem Ausstossen der ersten Flüssigkeit 51 bleibt die Lamelle 82 infolge Druckgleichheit in beiden Kammern verschlossen.

Sobald der Stützring 81 an der vorderen Zylinderkante 7 ansteht, wird durch verstärkten Druck in der hinteren Kammer 52 die Lamelle 82 selbsttätig geöffnet, so dass die Flüssigkeit 52 durch Bewegung des Kolbens 10 durch die Öffnung 83 ausfliesst.

In der Fig. 6 ist eine Draufsicht in axialer Richtung auf den Trennkolben 80 der Fig. 4 dargestellt. Gut sichtbar ist der Stützring 81, an dem die kreisförmige Lamelle 82 befestigt ist. Die Öffnung 83 der Lamelle ist durch den Verschlussteil 85 zapfenartig verschlossen.

Im folgenden soll das Vorgehen zum Füllen der Injektionsspritze 1 mit zwei Flüssigkeiten 51 und 52 erläutert werden.

Der leere Zylinder 2 wird in der Nähe der Austrittsöffnung 6 verschlossen und vollständig mit der nachinjizierten Flüssigkeit 52, beispielsweise physiologische Kochsalzlösung, gefüllt. Nun wird der Kolben 10 mit dem in ausgezogener Position aufgesetzten Trennkolben 30 langsam eingeführt. Die Flüssigkeit strömt durch den Spalt 34 zwischen Trennkolben 30 und Zylinderinnenwand 5 und füllt die hintere Kammer 62, wobei die leichte Konizität der Rückfläche 35 des Trennkolbens 30 und der Frontfläche 32 des Trennkolbens 30 die luftfreie Füllung erleichtern. Sobald der Kolben 10 so weit eingeführt ist, dass die Dichtung 12 des Kolbens 10 mit der Zylinderinnenwand 5 in Berührung kommt, erhöht sich der Einführungswiderstand stark. Nun wird der Zylinder 2 vorne wieder geöffnet und der Kolben 10 um etwa 1 cm vorgestossen und anschliessend der Zylinder 2 an einem Injektor angeschlossen. Anschliessend wird die vordere Kammer 61 durch Vorschieben des Kolbens 10 geleert und durch Zurückziehen Kontrastmittel aufgesogen.

## Patentansprüche

1. Injektionsspritze (1) zum aufeinanderfolgenden Einspritzen von zwei Flüssigkeiten (51, 52) in Blutgefässe lebender Körper, bestehend aus einem auf der einen Seite mit einem Befestigungsflansch (3) und auf der anderen Seite in der Nähe einer Austrittsöffnung (6) mit einem Katheteransatz (4) versehenen Zylinder (2), in welchem ein erster, an der Zylinder-Innenwand (5) flüssigkeitsabdichtender Kolben (10) und ein zweiter, die beiden Füssigkeiten separierender Kolben (30) relativ zueinander längsverschiebbar geführt sind, wobei die Kolben durch mit ihnen verbundenen und ineinandergesteckten Kolbenstangen betätigbar sind, dadurch gekennzeichnet, dass die mit dem einen Ende mit dem zweiten Kolben (30, 80) verbundene Kolbenstange als Distanzstange (70) ausgebildet und mit dem anderen Ende in einer axialen Bohrung (13) des ersten Kolbens (10) durch eine federelastische Haltevorrichtung (20) derart gehalten ist, dass nach dem Ausstossen der zwischen zweitem Kolben und der Austrittsöffnung befindlichen Flüssigkeit (51) durch das Zusammenwirken des zweiten Kolbens (30, 80) mit der Zylinderkante (7) des Zylinderbodens (8) die Haltevorrichtung (20) selbsttätig gelöst und dadurch der erste Kolben (10) relativ zu dem zweiten Kolben (30, 80) zum Ausstossen der zwischen erstem und zweitem Kolben befindlichen Flüssigkeit (52) verschiebbar ist.

2. Injektionsspritze nach Anspruch 1, dadurch gekennzeichnet, dass die der Austrittsöffnung (6) des Zylinders (2) zugewandte Kolben-Frontfläche (32) des mit geringem Spalt (34) zur Zylinder-Innenwand (5) ausgebildeten zweiten Kolbens (30) konisch oder konvex ausgebildet und ausgehend von der zylindrischen Mantelfläche (31) mit mehreren strahlenförmig verlaufenden Durchflusskanälen (33) versehen ist.

3. Injektionsspritze nach Anspruch 1, dadurch gekennzeichnet, dass als zweiter Kolben ein mit einer elastisch verformbaren Lamelle (82) versehener Kolben (80) vorgesehen ist, bei welchem die Lamelle (82) an einem an der Zylinder-Innenwand (5) verschiebbar geführten Stützring (81) befestigt und während dem Ausstossen der zwischen ihm und der Austrittsöffnung befindlichen Flüssigkeit (51) flüssigkeitsdicht schliesst und beim nachfolgenden Ausstossen der zwischen ihm und dem ersten Kolben befindlichen Flüssigkeit (52) selbsttätig eine zur Austrittsöffnung (6) des Zylinders (2) orientierte Öffnung (83) bildet.

**Claims**

1. Syringe (1) for the successive injection of two fluids (51, 52) into blood vessels of living bodies, comprising a cylinder (2) provided on one side with a securing flange (3) and on the other side, in the region of an outlet aperture (6), with a catheter attachment (4), in which cylinder a first plunger (10), fluid-tight on the cylinder inner wall (5), and a second plunger (30) separating the two fluids, are guided in longitudinally displaceable manner relatively to each other, wherein the plungers can be actuated by piston rods connected to them and inserted into each other, characterised in that the piston rod connected with one end to the second plunger (30, 80) is constructed as a spacer rod (70) and with the other end is held in an axial bore (13) of the first plunger (10) by means of a resilient holding device (20) in such a way that after the ejection of the fluid (51) located between the second plunger and the outlet aperture, through the co-operation of the second plunger (30, 80) with the cylinder edge (7) of the cylinder base (8), the holding device (20) is automatically released and hence the first plunger (10) is movable relatively to the second plunger (30, 80) to eject the fluid (52) located between the first and second plungers.

2. Syringe according to claim 1, characterised in that the plunger front face (32), facing the outlet aperture (6) of the cylinder (2), of the second plunger (30) constructed with a small gap (34) to the cylinder inner wall (5), is of conical or convex construction, and proceeding from the cylindrical casing surface (31) is provided with several discharge ducts (33) extending in radial manner.

3. Syringe according to claim 1, characterised in that there is provided as a second plunger a plunger (80) provided with a resiliently deformable lamella (82), in which plunger (80) the lamella (82) is secured on a support ring (81) movably guided on the cylinder inner wall (5) and, during the ejection of the fluid (51) located between it and the outlet aperture, closes in fluid-tight manner, and during the subsequent ejection of the fluid (52) located between it and the first plunger, automatically forms an aperture (83) directed towards the outlet aperture (6) of the cylinder (2).

**Revendications**

1. Seringue d'injection (1) pour injecter successivement deux liquides (51, 52) dans les vaisseaux sanguins d'un organisme vivant, comprenant d'un côté une bride de fixation (3) et de l'autre côté un cylindre (2) muni d'un prolongement pour cathéters (4) au voisinage d'un orifice de sortie (6), cylindre dans lequel sont susceptibles de coulisser longitudinalement et de manière relative un premier piston (10) appliqué de manière étanche aux liquides contre la paroi intérieure (5) du cylindre et un second piston (30) séparant les deux liquides, les deux pistons étant susceptibles d'être actionnés par des tiges de piston qui les relient et les traversent, caractérisée en ce que la tige de piston dont une extrémité est reliée au second piston (30, 80) est réalisée en forme de tige d'écartement (70), son autre extrémité traversant un perçage axial (13) du premier piston (10) en passant par un dispositif de fixation (20) élastique à ressort en étant maintenue de manière qu'après expulsion de liquide (51) se trouvant entre le second piston et l'orifice de sortie, la coopération du second piston (30, 80) et de l'arête (7) du fond (8) du cylindre déverrouille automatiquement le dispositif de fixation (20) et permet le coulissement du premier piston (10) par rapport au second piston (30, 80) pour expulser le liquide (52) compris entre le premier et le second piston.

2. Seringue d'injection selon la revendication 1, caractérisée en ce que la surface avant (32) du second piston (30) qui laisse un faible intervalle (34) par rapport à la paroi intérieure (5) du cylindre, surface avant (32) tournée vers l'orifice de sortie (6) du cylindre (2) est de forme conique ou convexe et comporte plusieurs canaux de passage (33) qui sont disposés de manière radiale en partant de la surface-enveloppe cylindrique (31).

3. Seringue d'injection selon la revendication 1, caractérisée en ce que le second piston est un piston (80) muni d'une lamelle (82) déformable élastiquement, la lamelle (82) étant fixée sur une bague d'appui (81) en étant coulissante sur la paroi intérieure (5) du cylindre et pendant l'expulsion du liquide (51) se trouvant entre ce second piston et l'orifice de sortie, cette lamelle est étanche aux liquides et lors de l'expulsion consécutive du liquide (52) se trouvant entre ce piston et le premier piston, cette lamelle forme automatiquement un orifice (83) orienté vers l'orifice de sortie (6) du cylindre (2).

FIG. 1

FIG. 2

FIG. 3

FIG. 4

FIG. 5

FIG. 6